# DEMANDE DE BREVET EUROPEEN

(11) **EP 0 522 971 A1**
(43) Date de publication de la demande: **13.01.1993**
(21) Numéro de dépôt: 92402006.8
(22) Date de dépôt: 10.07.1992
(51) Int. Cl.: C07D 471/04, A61K 31/435

(54) **Un isomère dextrogyre d'un dérivé de amino-2 naphtyridine et son procédé pour son préparation**

(30) Priorité: 12.07.1991 FR 9108829
(71) Demandeur: RHONE-POULENC RORER S.A., 92165 Antony Cédex (FR)
(72) Inventeur: David-Comte, Marie-Thérèse, F-94500 Chevilly Larue (FR)
(74) Mandataire: Pilard, Jacques

(57) **Abrégé**

L'isomère dextrogyre de Formule (I) et procédé de préparation de l'isomère dextrogyre du produit de formule (I) à partir d'un sel du produit racémique correspondant avec la cinchonine puis avec la cinchonidine.

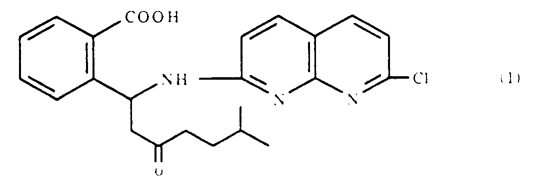

## Description

La présente invention concerne un nouveau procédé de préparation des isomères optiques du dérivé de l'amino-2 naphtyridine de formule :

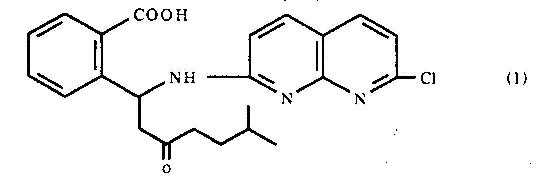

Plus particulièrement, la présente invention concerne la préparation de l'isomère dextrogyre du produit de formule (I) qui est utile pour préparer l'isomèredextrogyre du produit de formule : 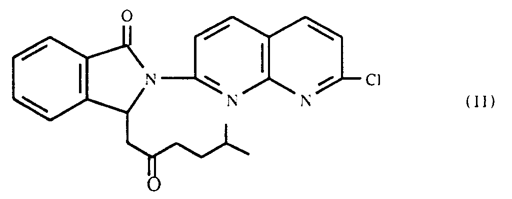
qui présente des propriétés anxiolytiques, hypnotiques, anticonvulsivantes, antiépileptiques et myorelaxantes remarquables.

Il a été montré que, pour le produit de formule (II), qui, avec des produits analogues, fait l'objet du brevet américain US 4 960 779, l'entité active ou eutomère est l'isomère dextrogyre (+)

Selon le brevet américain US 4 960 779, la séparation des isomères optiques du produit de formule (II) peut être effectuée par chromatographie sur phase chirale. Cependant, l'application industrielle de ce procédé n'est pas toujours de réalisation commode.

Selon l'invention, l'isomère dextrogyre du produit de formule (I) est obtenu en précipitant d'abord le sel du produit de formule (I) lévogyre avec la cinchonine, puis, après déplacement de l'isomère dextrogyre de son sel avec la cinchonine dans les eaux-mères de filtration du sel de l'isomère lévogyre avec la cinchonine, en précipitant le sel de l'isomère dextrogyre avec la cinchonidine.

L'isomère dextrogyre du produit de formule (I), après déplacement de son sel avec la cinchonidine, est transformé en isomère dextrogyre du produit de formule (II) par cyclisation.

Selon l'invention, la formation du sel de produit de formule (I) racémique avec la cinchonine est effectuée en opérant dans un solvant organique tel que l'éthanol dans lequel le sel est insoluble.

Le produit de formule (I) enrichi en isomère dextrogyre sous forme de sel soluble avec la cinchonine, qui est dans les eaux-mères de filtration, est déplacé de son sel après acidification au moyen d'un acide fort tel que l'acide chlorhydrique. Le produit enrichi en isomère dextrogyre traité par la cinchonidine dans un solvant organique tel que l'éthanol conduit à la précipitation du sel de l'isomère dextrogyre pur du produit de formule (I) avec la cinchonidine. L'isomère dextrogyre pur du produit de formule (I) est déplacé de son sel avec la cinchonidine au moyen d'un acide fort tel que l'acide chlorhydrique.

L'isomère dextrogyre du produit de formule (I) est cyclisé en eutomère du produit de formule (II) au moyen de chlorure de thionyle en opérant éventuellement en présence d'un agent de condensation tel que l'imidazole ou la pyridine dans un solvant organique tel que le chlorure de méthylène.

Le produit de formule (I) peut être obtenu par ouverture du cycle pyrrolinone d'un produit de formule (II) racémique en milieu basique.

Généralement, l'ouverture du cycle pyrrolinone est effectuée au moyen d'une base minérale à une température comprise entre 0 et 50°C et, de préférence, comprise entre 0 et 30°C.

Généralement, le procédé est mis en oeuvre en agitant une solution hydro-organique du produit de formule (II) en présence d'un excès de base minérale choisie parmie les hydroxydes et les carbonates ou bicarbonates de métaux alcalins ou alcalino-tereux. Il est particulièrement avantageux d'utiliser la soude comme base minérale et d'opérer dans un mélange eau-pyridine. Il est aussi possible d'effectuer la réaction en utilisant le mélange eau-dioxanne comme solvant.

Le produit de formule (I) peut aussi être obtenu par action d'une base minérale sur le produit de formule :

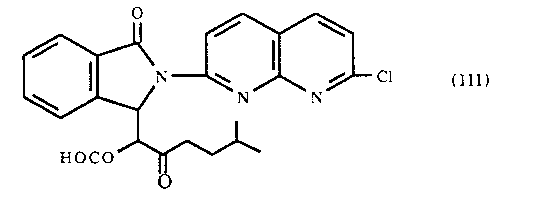

Généralement, on fait agir au moins deux équivalents de la base minérale choisie, de préférence, parmi la soude, la potasse, le carbonate de sodium ou le carbonate de potassium en opérant dans l'eau ou dans un milieu hydro-organique à une température comprise entre 0 et 50°C, de préférence, entre 0 et 30°C. Comme milieu hydro-organique, on utilise de préférence un mélange dioxanne-eau.

Le produit de formule (III) peut être obtenu par hydrolyse en milieu acide d'un produit de formule générale : 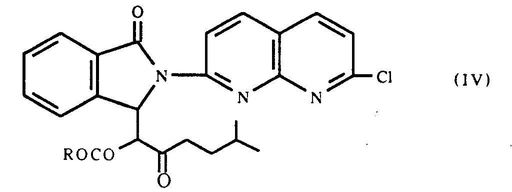
dans laquelle R représente un radical alkyle contenant 1 à 10 atomes de carbone en chaîne droite ou ramifiée.

Généralement, l'hydrolyse est effectuée au moyen d'un acide minéral fort tel que l'acide sulfurique concentré en opérant à une température comprise entre 0 et 50°C, de préférence voisine de 20°C.

Les produits de formule (III) et (IV) peuvent être obtenus dans les conditions décrites dans le brevet américain US 4 960 779.

Les exemples suivants illustrent l'invention.

### EXEMPLE 1

Dans un réacteur agité de 2 litres, on introduit 1450 cm3 d'éthanol à 95 % (v/v), 100 g de cinchonine et 145 g d'acide {[(chloro-7 naphtyridine-1,8 yl-2) amino]-1 méthyl-6 oxo-3 heptyl}-2 benzoïque. La suspension est chauffée à 40°C puis refroidie, en 3 heures 30 minutes, à 10°C. La suspension obtenue est filtrée. Le précipité est lavé par 2 fois 50 cm3 d'éthanol à 10°C puis séché à 60°C pendant 16 heures sous pression réduite (15 mm de mercure; 2,0 kPa). On obtient ainsi 99,3 g de sel de cinchonine et d'acide {[(chloro-7 naphtyridine-1,8 yl-2) amino]-1 méthyl-6 oxo-3 heptyl}-2 benzoïque sous forme d'un produit blanc dont les caractéristiques sont les suivantes :
- pouvoir rotatoire: [α]²⁰_{D} = +192,6° (c = 1 ; chlorure de méthylène)
- titre énantiomérique : 98,5 %

Dans un réacteur agité de 2 litres, on introduit 250 cm3 de N-méthylpyrrolidone et 50 g du sel obtenu précédemment. On ajoute, en 30 minutes, en maintenant la température à 20°C, 90 cm3 d'acide chlorhydrique N. On laisse pendant 1 heure à cette température puis on ajoute, en 1 heure, 660 cm3 d'eau distillée. La suspension obtenue est filtrée. Le précipité est lavé par 5 fois 100 cm3 d'eau puis séché à 60°C pendant 16 heures sous pression réduite (15 mm de mercure; 2,0 kPa).

On obtient ainsi 28,6 g d'acide (-)-{[(chloro-7 naphtyridine-1,8 yl-2) amino]-1 méthyl-6 oxo-3 heptyl}-2 benzoïque sous forme d'un produt blanc dont les caractéristiques sont les suivantes :
- pouvoir rotatoire : [α]²⁰_{D} = -227,4° (c = 1 ; chlorure de méthylène)
- titre énantiomérique : 99,6 %.

L'acide {[(chloro-7 naphtyridine-1,8 yl-2) amino]-1 méthyl-6 oxo-3 heptyl}-2 benzoique peut être préparé selon l'une des méthodes suivantes :
1) Dans un réacteur agité de 2 litres, on introduit à une température voisine de 20°C, 1400 cm3 de dioxanne et 20 g de (chloro-7 naphtyridine-1,8 yl-2)-2 (méthyl-5 oxo-2 hexyl)-3 isoindolinone-1. On additionne en 5 minutes 244 cm3 d'une solution aqueuse de soude N. On laisse réagir pendant 4 jours à une température inférieure à 30°C.
   Le dioxanne est éliminé par distillation sous pression réduite (40 mm de mercure ; 5,3 kPa) à une température inférieure à 30°C. Pendant la distillation, on ajoute 100 cm3 d'eau distillée.
   On élimine un produit insoluble par filtration à 20°C. Ce produit est lavé par 3 fois 50 cm3 d'eau distillée et est éliminé. Les phases aqueuses réunies sont acidifiées par addition, en 3 heures, de 48 cm3 d'acide chlorhydrique 5N à une température de 20°C. Le pH de la suspension est alors voisin de 3,5.
   Après filtration de la suspension, le précipité est lavé par 6 fois 100 cm3 d'eau distillée puis séché sous pression réduite (15 mm de mercure; 2,0 kPa) à 60°C pendant 16 heures.
   On obtient ainsi 14,3 g d'acide {[(chloro-7 naphtyridine-1,8 yl-2) amino]-1 méthyl-6 oxo-3 heptyl}-2 benzoïque sous forme d'un produit blanc dont le temps de rétention est de 4,8 minutes par chromatographie liquide à haute performance en utilisant une colonne de 25 cm de longueur et de 0,46 cm de diamètre avec comme phase stationnaire du "Lichrospher O.D.S. 5 µm" et comme phase mobile un mélange de 200 cm3 de tampon phosphate pH 3 25 mM, de 560 cm3 d'acétonitrile et de 240 cm3 de méthanol au débit de 0,8 cm3/minute.
   La (chloro-7 naphtyridine-1,8 yl-2)-2 (méthyl-5 oxo-2 hexyl)-3 isoindolinone-1 peut être préparé selon la méthode décrite dans le brevet américain US 4 960 779.
2) Dans un réacteur agité de 1 litre, on introduit à une température voisine de 20°C, 20 g de (chloro-7 naphtyridine-1,8 yl-2)-2 (méthyl-5 oxo-2 hexyl)-3 isoindolinone-1, 400 cm3 de pyridine et 60 cm3 d'une solution aqueuse de soude 2N. On laisse réagir pendant 23 heures puis on distille la pyridine sous pression réduite (15 mm de mercure) à une température inférieure à 20°C. On ajoute 500 cm3 d'eau distillée. Un insoluble est séparé par filtration. La phase aqueuse est acidifiée à pH = 3,8 par addition de 40 cm3 d'acide chlorhydrique 4N. La suspension est filtrée, le précipité est lavé par 5 fois 140 cm3 d'eau distillée puis séché pendant 16 heures sous pression réduite (15 mm de mercure; 2,0 kPa) à 60°C.
   On obtient ainsi 19,2 g d'acide {[(chloro-7 naphtyridine-1,8 yl-2) amino]-1 méthyl-6 oxo-3 heptyl}-2 benzoïque sous forme d'un produit blanc dont le temps de rétention par chromatographie liquide à haute performance est de 4,8 minutes dans les conditions décrites précédemment.
3) Une suspension de 30 mg d'acide [(chloro-7 naphtyridine-1,8 yl-2)-2 oxo-3 isoindolinyl-1]-2 méthyl-6 oxo-3 heptanoïque dans 4,7 cm3 d'eau distillée et 1,32 cm3 d'une solution aqueuse de soude 0,1N est agitée à une température voisine de 20°C pendant 72 heures. On élimine un produit insoluble par filtration et le filtrat est acidifié jusqu'à pH = 2 par addition d'une solution aqueuse d'acide chlorhydrique 0,1N. Le précipité obtenu est séparé par filtration, lavé à l'eau et séché à l'air. On obtient ainsi 10 mg d'acide {[(chloro-7 naphtyridine-1,8 yl-2) amino]-1 méthyl-6 oxo-3 heptyl}-2 benzoïque dont les caractéristiques sont identiques à celles du produit obtenu précédemment.

L'acide [(chloro-7 naphtyridine-1,8 yl-2)-2 oxo-3 heptanoïque peut être préparé de la façon suivante :
Une solution de 23 g de [(chloro-7 naphtyridine-1,8 yl-2)-2 oxo-3 isoindolinyl-1]-2 méthyl-6 oxo-3 heptanoate d'éthyle dans 235 cm3 d'acide sulfurique à 98 %, est agitée pendant 20 heures à une température voisine de 20°C puis versée dans 1,5 kg de glace. Le précipité obtenu est séparé par filtration, lavé à l'eau jusqu'à pH = 6 et séché à l'air. Le solide obtenu est repris par 3,8 litres d'eau distillée et 480 cm3 d'une solution aqueuse de soude 0,1N. Le produit insoluble est séparé par filtration, le filtrat est acidifié jusqu'à pH = 3 par addition d'une solution aqueuse d'acide chlorhydrique 0,1N. Le précipité obtenu est séparé par filtration, lavé à l'eau distillée puis à l'oxyde d'isopropyle et séché à 20°C sous pression réduite (0,07 kPa). On obtient ainsi 9,2 g d'acide [(chloro-7 naphtyridine-1,8 yl-2)-2 oxo-3 isoindolinyl-1]-2 méthyl-6 oxo-3 heptanoïque fondant à 176°C.

Le [(chloro-7 naphtyridine-1,8 yl-2)-2 oxo-3 isoindolinyl-1]-2 méthyl-6 oxo-3 heptanoate d'éthyle peut être obtenu par la méthode décrite dans le brevet américain US 4 960 779.

### EXEMPLE 2

Dans un réacteur agité de 1 litre, on introduit à une température de 20°C, 400 cm3 de chlorure de méthylène, 20 g du produit obtenu précédemment et 21,8 g d'imidazole. A l'aide d'une seringue, on ajoute, en 10 minutes, 7 cm3 de chlorure de thionyle. La suspension est chauffée au reflux pendant 30 minutes puis est refroidie à 20°C et lavée par 2 fois 200 cm3 d'eau distillée. La solution lavée est concentrée à la moitié de son volume puis on ajoute 450 cm3 d'éthanol absolu. La distillation sous pression atmosphérique est poursuivie jusqu'à ce que la température des vapeurs soit de 78°C. On ajoute 1 g de noir décolorant puis maintient pendant 1 heure à 70°C. La suspension est filtrée. Le précipité est lavé par 50 cm3 d'éthanol à 75°C. Le filtrat et le lavage sont réunis. Après refroidissement en 2 heures à 15°C, la suspension est filtrée. Le précipité est lavé par 3 fois 35 cm3 d'éthanol puis séché à 60°C pendant 16 heures sous pression réduite (15 mm de mercure; 2,0 kPa).

On obtient ainsi 16,7 g de (-)-(chloro-7 naphtyridine-1,8 yl)-2 (méthyl-5 oxo-2 hexyl)-3 isoindolinone-1 sous forme d'un produit cotonneux blanc dont les caractéristiques sont les suivantes :
- pouvoir rotatoire: [α]²⁰_{D} = -132° (c = 1 ; chlorure de méthylène)
- titre énantiomérique : 100 %

### EXEMPLE 3

Dans un réacteur de 2 litres, on introduit 1274,3 g de liqueurs éthanoliques (correspondant au filtrat de sel de cinchonine obtenu précédemment puis addition du lavage éthanolique). A 20°C, on ajoute 260 cm3 d'une solution aqueuse d'acide chlorhydrique N. Après 15 minutes d'agitation, on ajoute 650 cm3 d'eau distillée. La solution est concentrée sous pression réduite (25 mm de mercure ; 3,3 kPa) à une température inférieure à 30°C pour éliminer l'éthanol. La suspension est ensuite filtrée. Le précipité est lavé par 6 fois 100 cm3 d'eau puis séché à 60°C pendant 16 heures sous pression réduite (15 mm de mercure; 2,0 kPa).

On obtient ainsi 79,6 g d'un produit blanc constitué majoritairement de l'isomère dextrogyre de l'acide {[(chloro-7 naphtyridine-1,8 yl-2) amino]-1 méthyl-6 oxo-3 heptyl}-2 benzoïque dont les caractéristiques sont les suivantes :
- pouvoir rotatoire: [α]²⁰_{D} = +160° (c = 1 ; chlorure de méthylène)
- titre énantiomérique : 73,2 %

Dans un réacteur de 1 litre, on introduit 78,5 g du produit obtenu précédemment, 54,3 g de cinchonidine et 700 cm3 d'éthanol à 95 % (v/v). La solution est chauffée à reflux puis refroidie en 3 heures à une température de 10°C. Un produit cristallise. La suspension est filtrée. Le précipité est lavé avec 2 fois 50 cm3 d'éthanol à 95 % puis séché à 60°C pendant 16 heures sous pression réduite (15 mm de mercure; 2,0 kPa).

On obtient ainsi 92,8 g de sel de cinchonidine de l'acide (+)-{[(chloro-7 naphtyridine-1,8 yl-2) amino]-1 méthyl-6 oxo-3 heptyl}-2 benzoïque sous forme d'un produit blanc dont les caractéristiques sont les suivantes :
- pouvoir rotatoire: [α]²⁰_{D} = -137,7° (c = 1 ; chlorure de méthylène)
- titre énantiomérique : 100 %

Dans un réacteur de 1 litre, on dissout 50 g du sel de cinchonidine obtenu précédemment dans 250 cm3 de N-méthylpyrrolidone. On ajoute, en 30 minutes, 90 cm3 d'acide chlorhydrique N en maintenant la température inférieure à 20°C. Après 15 minutes d'agitation à 20°C, on ajoute, en 1 heure, 600 cm3 d'eau distillée. La suspension obtenue est filtrée. Le précipité obtenu est lavé par 5 fois 100 cm3 d'eau distillée puis séché à 60°C pendant 16 heures sous pression réduite (15 mm de mercure ; 2,0 kPa).

On obtient ainsi 29,7 g d'acide (+)-{[(chloro-7 naphtyridine-1,8 yl-2) amino]-1 méthyl-6 oxo-3 heptyl}-2 benzoïque sous forme d'un produit blanc dont les caractéristiques sont les suivantes :
- pouvoir rotatoire : [α]²⁰_{D} = 222,8° (c = 1 ; chlorure de méthylène)
- titre énantiomérique : 100 %

### EXEMPLE 4

Dans un réacteur de 1 litre, on dissout 20 g de l'acide dextrogyre obtenu précédemment et 21,8 g d'imidazole dans 400 cm3 de chlorure de méthylène. A une température de 20°C, on introduit, à l'aide d'une seringue, 7 cm3 de chlorure de thionyle. La suspension est chauffée au reflux pendant 30 minutes, est ensuite refroidie à 20°C puis lavée 2 fois par 200 cm3 d'eau distillée. La solution est concentrée, sous pression atmosphérique, à la moitié de son volume puis on ajoute 450 cm3 d'éthanol absolu. La distillation du chlorure de méthylène est poursuivie jusqu'à ce que la température des vapeurs atteigne 78°C. On ajoute alors 1 g de noir décolorant puis laisse une heure à 78°C. La suspension est filtrée. Le précipité est lavé par 50 cm3 d'éthanol absolu à 75°C. Le filtrat et les lavages sont réunis puis refroidis à 15°C en 2 heures. La suspension est filtrée. Le précipité est lavé par 3 fois 35 cm3 d'éthanol absolu à 15°C puis séché à 60°C pendant 16 heures sous pression réduite (15 mm de mercure ; 2,0 kPa). On obtient ainsi 16,8 g de (+)-(chloro-7 naphtyridine-1,8 yl)-2 (méthyl-5 oxo-2 hexyl)-3 isoindolinone-1 sous forme d'un produit cotonneux blanc dont les caractéristiques sont les suivantes :
- pouvoir rotatoire: [α]²⁰_{D} = +132° (c = 1 ; chlorure de méthylène)
- titre énantiomérique : 98,8 %.

## Revendications

**1 -** Procédé de préparation de l'isomère dextrogyre du produit de formule : 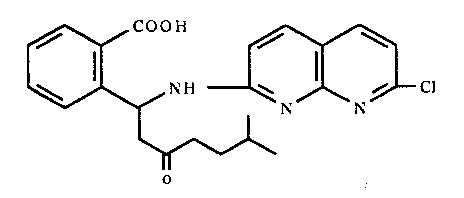 caractérisé en ce que l'on forme un sel insoluble du produit racémique correspondant avec la cinchonine, le sépare par filtration, déplace le produit enrichi en isomère dextrogyre de son sel avec la cinchonine, précipite le sel de l'isomère dextrogyre pur avec la cinchonidine et enfin libère l'isomère dextrogyre de son sel avec la cinchonidine.

**2 -** Procédé selon la revendication 1 caractérisé en ce que le sel du produit racémique avec la cinchonine est précipité dans un solvant organique tel que l'éthanol.

**3 -** Procédé selon la revendication 1 caractérisé en ce que le produit enrichi en isomère dextrogyre est séparé de son sel avec la cinchonine au moyen d'un acide fort tel que l'acide chlorhydrique.

**4 -** Procédé selon la revendication 1 caractérisé en ce que le sel du produit enrichi en isomère dextrogyre avec la cinchonidine est précipité dans un solvant organique tel que l'éthanol.

**5 -** Procédé selon la revendication 1 caractérisé en ce que l'isomère dextrogyre pur est déplacé de son sel avec la cinchonidine au moyen d'un acide fort tel que l'acide chlorhydrique.

**6 -** L'isomère dextrogyre du produit de formule : 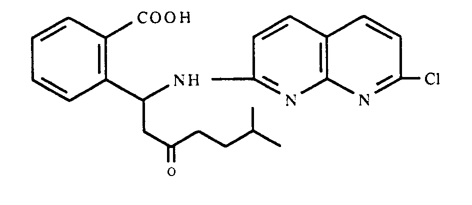 lorsqu'il est obtenu selon le procédé de l'une des revendications 1 à 5.

**7 -** Utilisation du produit selon la revendication 6 pour la préparation de l'isomère dextrogyre du produit de formule :
